# EUROPEAN PATENT SPECIFICATION

(11) **EP 4 426 311 B1**
(45) Date of publication and mention of the grant of the patent: **29.04.2026**
(21) Application number: 22813502.6
(22) Date of filing: 02.11.2022
(51) Int. Cl.: A61K 31/69, A61K 45/06, A61P 33/06

(54) **BOROMYCIN AS GAMETOCYTOCIDAL COMPOUND**
BOROMYCIN ALS GAMETOZYTOZIDE VERBINDUNG
BOROMYCINE COMME COMPOSÉ GAMÉTOCYTOCIDAL

(30) Priority: 02.11.2021 EP 21205968
(43) Date of publication of application: 11.09.2024
(73) Proprietor: Eberhard Karls Universität Tübingen (Medizinische Fakultät), 72074 Tübingen (DE)
(72) Inventor: HELD, Jana, 72074 Tübingen (DE); PESSANHA DE CARVALHO, Lais, 72070 Tübingen (DE); GRÖGER OTERO, Sara, 72070 Tübingen (DE); KREMSNER, Peter G., 72074 Tübingen (DE)
(74) Representative: Witte, Weller & Partner Patentanwälte mbB
(86) International application number: PCT/EP2022/080568
(87) International publication number: WO 2023/078941

(56) References cited:
- WO-A1-2018/106966
- WO-A1-2019/198825
- GB-A- 2 012 167
- US-A- 3 769 418
- US-A- 3 864 479
- DOMINIQUE LAURENT ET AL: "Antiplasmodial Marine Natural Products in the Perspective of Current Chemotherapy and Prevention of Malaria. A Review", MARINE BIOTECHNOLOGY, SPRINGER-VERLAG, NE, vol. 8, no. 5, 30 March 2006 (2006-03-30), pages 433 - 447, XP019431941, ISSN: 1436-2236, DOI: 10.1007/S10126-006-6100-Y
- SHIMIZU YOHEI ET AL: "Aplasmomycin and boromycin are specific inhibitors of the futalosine pathway", THE JOURNAL OF ANTIBIOTICS, NATURE PUBLISHING GROUP UK, LONDON, vol. 71, no. 11, 8 August 2018 (2018-08-08), pages 968 - 970, XP036623833, ISSN: 0021-8820, [retrieved on 20180808], DOI: 10.1038/S41429-018-0087-2
- DE CARVALHO LAÍS PESSANHA ET AL: "Boromycin has Rapid-Onset Antibiotic Activity Against Asexual and Sexual Blood Stages of Plasmodium falciparum", FRONTIERS IN CELLULAR AND INFECTION MICROBIOLOGY, vol. 11, 14 January 2022 (2022-01-14), XP055908510, DOI: 10.3389/fcimb.2021.802294

## Description

The present invention relates to a new compound for the inhibition of the transmission of malaria in humans, a pharmaceutical composition comprising said compound, and a method for the inhibition of the transmission of malaria in humans comprising the administration of said compound.

The references to the methods of treatment of this description are to be interpreted as references to compounds or pharmaceutical compositions of the present invention for use in a method for treatment of the human body by therapy.

### FIELD OF THE INVENTION

The invention relates to the field of pharmaceutically active compounds, more particular to the field of anti-malaria compounds.

### BACKGROUND OF THE INVENTION

Malaria remains one of the world's most serious public health problems, especially in tropical and subtropical areas, with approximately 229 million cases and 409,000 deaths in 2019. Intracellular parasites of the genus *Plasmodium* cause the disease among which six species can infect humans. These include *P*. *falciparum* as the most virulent species and *P*. *knowlesi*, described originally as a simian parasite, which can cause severe malaria in humans. Clinical symptoms of malaria appear during the replication of parasites in the erythrocytic cycle (lasting 24 h for *P*. *knowlesi* and 48 h for *P*. *falciparum*) in the human host. These symptoms can develop into life-threatening complications such as severe anemia, liver and kidney failure and cerebral malaria if not treated properly.

*Plasmodium* parasites have a complex life cycle that includes mosquito and vertebrate hosts. During each replication cycle, a small number of asexual parasites starts a different genetic program and develops into the transmissible sexual form, the gametocytes.

Currently, the mainstay malaria treatments are artemisinin-based combination therapies (ACT). Artemisinin and its derivatives are fast-acting and rapidly reduce the asexual parasite load but have a short half-life. They are combined to a slow-acting partner drug with longer half-life to clear remaining parasites, and to protect artemisinin against resistances. Nonetheless, reports of *P*. *falciparum* with delayed removal after artesunate or ACT treatment are accumulating, especially from the Greater Mekong Region where also resistances to the partner drugs are present. This poses a threat to the existing malaria treatment options. In addition, the current antimalarial drugs are inactive against mature *P*. *falciparum* (stage IV and V) gametocytes, favoring the transmission of the parasite.

The interest in using antibiotics for malaria treatment appeared due to the emergence of resistant parasites to the former mainstay drug chloroquine. Tetracyclines were the first antibiotics to treat uncomplicated malaria that dates back to the 1950s. Currently, doxycycline and the combination of sulfadoxine-pyrimethamine are used for malaria prevention of travelers and risk groups in malaria-endemic regions, respectively. In addition, clindamycin combined with quinine is used to treat pregnant women during the first trimester or combined with artesunate or quinine to treat uncomplicated malaria when an ACT is unavailable.

Tetracyclines and macrolides, both naturally produced by *Streptomyces*, have shown promising antiplasmodial activities. Most antibiotics with antiplasmodial activity target the apicoplast; a plastid-like organelle derived from endosymbiotic bacteria responsible for the biosynthesis of isoprenoid precursors. Antibiotics can be fast or slow-acting compounds, depending on whether they target the treated parasites or act on their progeny, causing the so-called delayed death effect. Commonly, tetracyclines belong to the latter group, but novel tetracyclines have shown superior antiplasmodial activity. Some macrolides such as ivermectin, borrelidin, and kitasamycin (also called leucomycin) were already described as fast-acting: they inhibit parasite growth during the first cycle *in vitro* and *in vivo* at low nanomolar concentrations but the molecular targets are not known.

Unfortunately, primaquine is the only approved drug active against gametocytes, therefore allowing the inhibition of the transmission of the malaria parasite between humans; see Sinden. Developing transmission-blocking strategies for malaria control. PLoS Pathog. 533 (2017) 13:1-12. However, its use is restricted mainly due to its haemolysis-inducing effect in patients with glucose-6-phosphate dehydrogenase deficiency (Wadi et al. Recent advances in transmission-blocking drugs for malaria elimination. Future Med Chem. (2019) 11:3047-88), and additionally it has to be metabolized by a liver enzyme (CYP2D6) to be active. Compounds with multi-stage activities and novel modes of action are especially desired for the next generation of antimalarial compounds.

Against this background it is an object underlying the invention to provide for a new compound which cannot only be used for the treatment of an acute illness from malaria but for controlling and eliminating malaria. Especially, such a compound is to be provided which allows the inhibition of the transmission of malaria and/or the malaria parasite in humans.

### SUMMARY OF THE INVENTION

The present invention is defined in the appended claims. Any embodiments which do not fall under the scope of the claims are not the subject of the present invention. The object is met by the provision of boromycin for use in the inhibition of the transmission of malaria in humans.

This object is also met by the provision of boromycin for use as a gametocytocidal compound.

Furthermore, this object is also met by the provision of a pharmaceutical composition for use in the inhibition of transmission of malaria in humans comprising boromycin and a pharmaceutically acceptable carrier.

According to the invention, "inhibition of the transmission" means that the transfer of the malaria parasite from the human being having received said boromycin to another human being is reduced or even prevented, said reduction or prevention is in comparison to the transfer from a reference human being, not having received said boromycin, to another human being. In an embodiment of the invention the inhibition of the transmission refers to an inhibitory activity of said boromycin against the developmental stage of the malaria parasite, which is responsible for the transmission to another host. In an embodiment of the invention said developmental stage is a gametocyte.

According to the invention, a "gametocytocidal compound" refers to an active agent which is destructive to gametocytes, i.e. the developmental stage of the malaria parasite which is responsible for the transmission to another host.

"Pharmaceutically acceptably carriers" are well known to the skilled person. They allow a proper formulation of the boromycin and serve to improve the selectivity, effectiveness, and/or safety of drug administration. Pharmaceutically acceptable carriers include, without being limited thereto, solvents, fillers, binders, lubricants, stabilizers, surfactants, suspensions, thickeners, emulsifiers, preserving agents, liposomes, micelles, microspheres, nanoparticles, etc. suitable for the particular form of dosage. Except for cases, when the medium of conventional carriers is incompatible with the active ingredient, for example, upon occurrence of any undesirable biological effects or other adverse interactions with any other ingredient(s) of the pharmaceutical composition, the use of such compositions falls within the scope of this invention. Materials that can serve as pharmaceutically acceptable carriers include, but are not limited to, monosaccharides and oligosaccharides, as well as derivatives thereof; malt, gelatin; talc; excipients such as: cocoa butter and suppository waxes; oils, such as peanut oil, cottonseed oil, safflower oil, sesame oil, olive oil, corn oil and soybean oil; glycols such as propylene glycol; esters, such as ethyl oleate and ethyl laurate; agar; buffering agents, such as magnesium hydroxide and aluminium hydroxide; alginic acid; pyrogen-free water; isotonic saline, Ringer's solution; ethyl alcohol and phosphate buffer solutions. In addition, the composition may contain other non-toxic compatible lubricants, for example sodium lauryl sulfate and magnesium stearate, as well as coloring agents, parting liquids, film formers, sweeteners, flavoring additives and flavorants, preserving agents and antioxidants.

The phrase "pharmaceutically acceptable" refers to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio. Suitable pharmaceutical carriers or excipients as well as pharmaceutical necessities for use in pharmaceutical formulations are described in Remington - The Science and Practice of Pharmacy, 23rd edition, 2020, a well-known reference text in this field, and in the USP/NF (United States Pharmacopeia and the National Formulary). Other sources are also available to one of ordinary skill in the art.

The pharmaceutical composition as well as the method to be explained below of the present invention may be utilized to treat a human being in need thereof.

"Boromycin" (CAS number: 34524-20-4, molecular mass: 879.89 mol⁻¹, formula: C₄₅H₇₄BNO₁₅) is a bacteriocidal polyether-macrolide antibiotic. It was first isolated from a *Streptomyces antibioticus* found in a soil sample from Ivory Coast and was first described in 1967. It is notable for being the first natural product found to contain the element boron; see Dunitz et al. Structure of Boromycin. Helv Chim Acta. (1971) 54:1709-13.

According to the invention, boromycin includes pharmaceutically acceptable salts thereof, as well as derivatives of boromycin and/or its pharmaceutically acceptable salts.

The findings of the inventors were surprising and could not have been suspected. So far, boromycin has been described in the art in relation with different activities.

Boromycin has so far been described a potent effect against Gram-positive bacteria *in vitro*; see Moreira et al. Boromycin kills Mycobacterial persisters without detectable resistance. Front in Microbiol. (2016) 7:199. It was also shown to be active against HIV; see Kohno et al. Boromycin, an Anti-HIV antibiotic. Biosci Biotechnol Biochem. (1996) 60:1036-7. Furthermore, activities against *Babesia* (Prelog et al. (1973). Antibiotic A 28829. U.S. patent no. 584,215. Ardsley, New York: Ciba-Geigy Corporation) and other pathogens (Abenoja et al. Boromycin has potent anti-Toxoplasma and anti-Cryptosporidium activity. Antimicrob Agents Chemother. (2021) 65:e01278-20) have been described.

U.S. patent no. 3,769,418 discloses an antibiotic named "A 28829" formed by cultivating *Streptomyces antibioticus* A 28829, said antibiotic is described as having an activity against *Plasmodium berghei* and *Plasmodium gallinaceum* which may infect rodents and poultry but no humans.

The inventors found that boromycin is a highly active and fast acting antibiotic against *Plasmodium* species which may cause malaria in humans. Importantly, they found that boromycin cannot only be used as a conventional anti-malaria substance but to prevent or reduce the transmission of the *Plasmodium* species to other subjects due to its surprising gametocytocidal activity.

In the investigation of the inventors, boromycin showed a high activity and a fast onset of action, a desired property of antimalarials to quickly reduce parasite load and avoid the disease progression to the severe form. It also showed an excellent selectivity and a favorable toxicity profile.

Boromycin's *in vitro* activity against laboratory strains showed superior activity as it was active at low nanomolar and even at picomolar concentrations. No cross-resistance with chloroquine/multi-resistant strains was seen.

The inventors additionally evaluated the activity of boromycin against mature gametocytes and could see a very potent activity superior to the positive control and similarly active as a reference compound. Drugs with transmission-blocking activity are of special interest and would be a great asset for controlling and eliminating malaria.

The object underlying the invention is herewith completely met.

In an embodiment of the invention said malaria is caused by *Plasmodium falciparum*.

The inventors have found in an experimental set-up that boromycin is of particular activity against a transmission of malaria caused by *Plasmodium falciparum.* This finding is important because *Plasmodium falciparum* is the most virulent intracellular malaria-causing parasite of the genus *Plasmodium,* which can infect humans. It is mainly found in Africa and is responsible for most malaria cases and deaths.

In another embodiment of the invention said malaria is caused by *Plasmodium knowlesi*.

In another experimental set-up the inventors were able to demonstrate activity against the species of *Plasmodium knowlesi*. *Plasmodium knowlesi*, described originally as a simian parasite, was recognized as a human malaria parasite only in 2000. Although *Plasmodium knowlesi* is prevalent only in a restricted area in South-East Asia and not responsible for many infections when regarded globally, it can cause severe malaria in humans and is phylogenetically closer to other not cultivable human *Plasmodi*um species. The invention provides a remedy for this.

While in one embodiment of the pharmaceutical composition according to the invention said boromycin is the only active ingredient, in another embodiment said pharmaceutical composition comprises an additional active ingredient.

Said latter measure has the advantage that the addition of an additional active ingredient may result in an action amplification of the boromycin or even a synergistic effect. E.g., the additional active ingredient may be another anti-malaria compound.

Another subject-matter of the invention relates to a method for the inhibition of the transmission of malaria in humans comprising the administration of the boromycin according to the invention or of the pharmaceutical composition according to the invention into a human suffering from or suspected of having malaria or of being infected by the parasite.

The feature, characteristics, advantages and embodiments disclose for boromycin according to the invention or of the pharmaceutical composition according to the invention apply to the method according to the invention *mutatis mutandis*.

It is to be understood that the before-mentioned features and those to be mentioned in the following cannot only be used in the combination indicated in the respective case, but also in other combinations or in an isolated manner without departing from the scope of the invention.

The invention is now further explained by means of embodiments resulting in additional features, characteristics and advantages of the invention. The embodiments are of pure illustrative nature and do not limit the scope or range of the invention. The features mentioned in the specific embodiments are general features of the invention which are not only applicable in the specific embodiment but also in an isolated manner and in the context of any embodiment of the invention.

The invention is now described and explained in further detail by referring to the following non-limiting examples and figures.

### BRIEF DESCRIPTION OF THE FIGURES

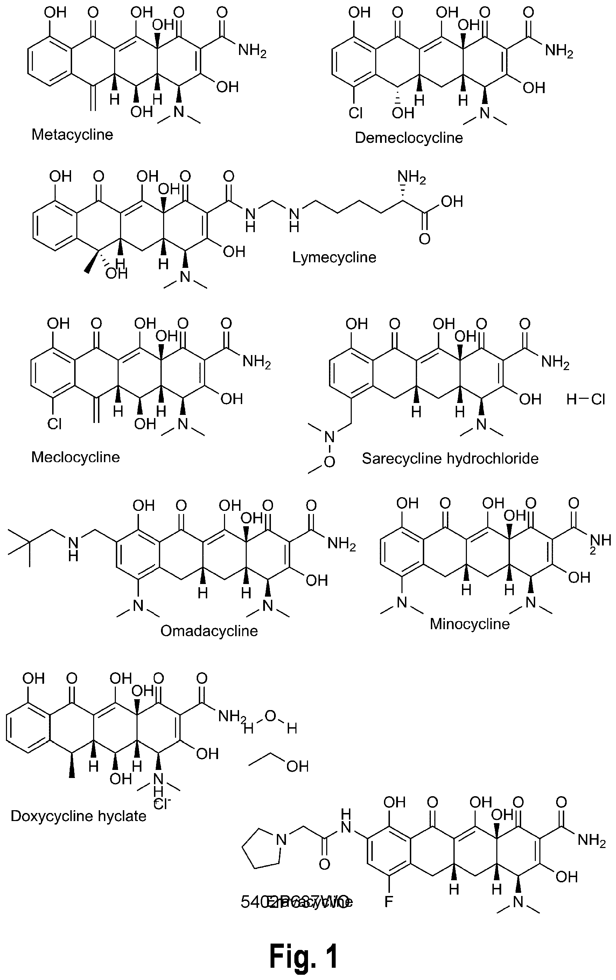

- Fig. 1:: Chemical structures of tetracyclines tested by the inventors.
- Fig. 2:: Chemical structures of macrolides tested by the inventors.
- Fig. 3:: Representative micrographies of *Plasmodium falciparum* infected erythrocytes stained with Giemsa: (A) untreated ring stage parasites, (B) strain 3D7 treated with 1 nM of boromycin for 6 h and (C) strain Dd2 treated with 1 nM of boromycin for 6 h. Arrows: parasites. Scale bar: 10 µm.
- Fig. 4:: Activity of boromycin, and the positive controls epoxomicin and methylene blue against mature (stage IV-V) *Plasmodium falciparum* gametocytes treated for 48 h. Boromycin showed a more potent activity than methylene blue and a comparable activity to epoxomicin. All experiments have been done at least 3 times in duplicate.

### EXAMPLES

### 1. Material and methods

### 1.1 Chemicals

All tested drugs were first dissolved in their respective solvents and subsequently further diluted in complete medium to have the desired final concentration (see Table 1).

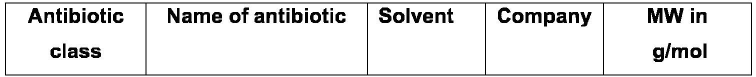

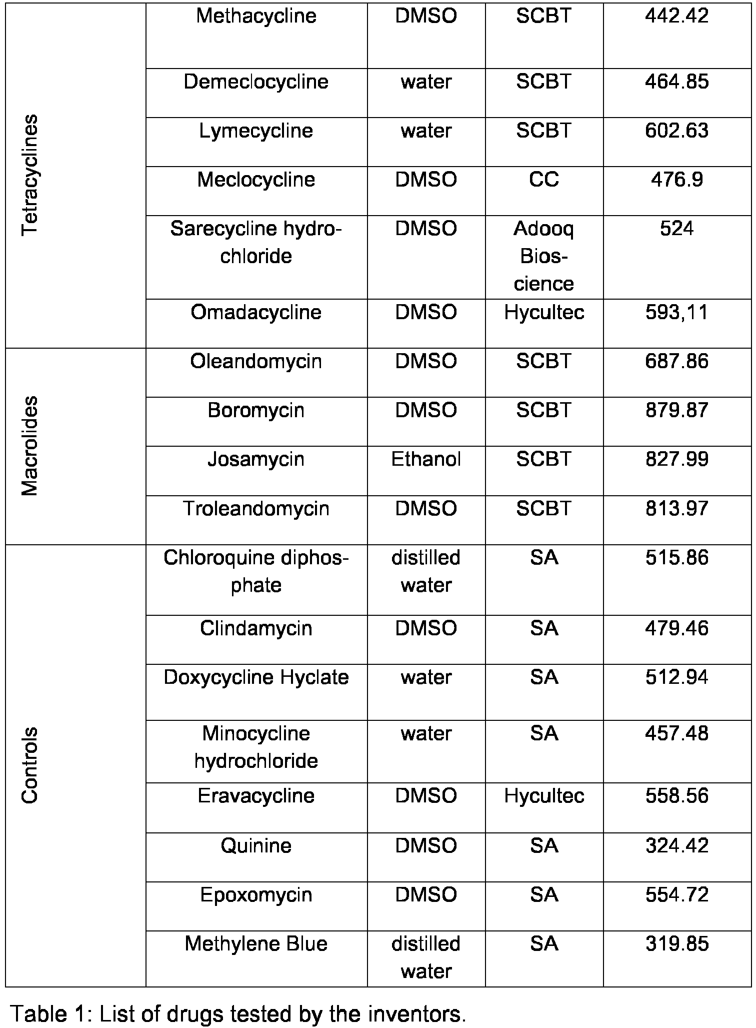

The concentration of the solvent did not interfere with parasite growth.

### 1.2 Parasite in vitro culture

### 1.2.1 Plasmodium falciparum asexual stages

*Plasmodium falciparum* laboratory strains 3D7 (chloroquine-sensitive, provided by BEI resources, MRA-102), Dd2 (multidrug-resistant, provided by BEI resources, MRA-150), K1 (multidrug-resistant, provided by BEI resources, MRA-159), 7G8 (chloroquine resistant, provided by BEI resources, MRA-154) and NF-54 (chloroquine sensitive, provided by Sanaria, a vigorous gametocyte producer), and *Plasmodium knowlesi* laboratory strain A1-H.1 (kindly provided by Robert Moon of LSHTM, London, UK) were cultivated *in vitro* as described before. Briefly, the parasites were maintained in complete culture medium consisting of RPMI-1640 (Sigma-Aldrich) supplemented with 1M HEPES N-2-hydroxyethylpiperazine-N-2-ethane sulfonic acid (HEPES) solution (2.4% v/v) (Sigma-Aldrich), 200 mM L-glutamine (Gibco), 50 µg/ml gentamicin (Gibco), and 10% of AlbuMax II solution containing RPMI, HEPES, NaHCO₃, D-Glucose and 0.5% wt/vol of albumin, 0.4 g of hypoxanthine at 2.5% hematocrit. The cultures were maintained at 5% CO₂, 5% O₂, at 37°C, with a change of medium every two days. *Plasmodium falciparum* parasites were synchronized before assays through magnetic column separation.

### 1.2.2 Plasmodium knowlesi asexual stages

*Plasmodium knowlesi* was cultivated as described for *Plasmodium falciparum*, with minor modifications. Parasites were cultivated in complete culture medium as detailed above supplemented with 5% human AB serum. Shortly before the growth inhibition assays, *Plasmodium knowlesi* were synchronized by density centrifugation with Nycodenz solution (Progen) to recover mature schizonts as described before. The Nycodenz solution was prepared according to the manufacturer's instruction and kept protected from the light at 4°C and warmed before using.

The asexual growth inhibition assays were performed with 3D7 and A1-H.1, while NF-54 was used for gametocyte assays.

### 1.2.3 Plasmodium falciparum sexual stages

*Plasmodium falciparum* gametocyte culture was performed as described previously with minor modifications. The gametocyte culture was initiated with synchronized ring-stage NF54 parasites at 6% hematocrit and 0.3% parasitemia. Complete culture medium (as described above) was supplemented with 5% human serum and changed daily without parasite dilution for 14 days. The medium was doubled when parasitemia reached 5%, and 50-mM N-acetyl-d-glucosamine (MP Biomedicals GmbH) was added between day 11 and day 14 to remove asexual stages. On day 15, mature gametocytes were purified with a Nycodenz and magnetic column separation (MACS) to remove erythrocytes and concentrate the gametocyte population.

### 1.3 Growth inhibition assays with asexual parasites

Growth inhibition assays of asexual stages of *Plasmodium falciparum* were performed as described before. Briefly, the drugs (see Table 1) were distributed in a 3-fold serial dilution in 96-well plates. Synchronized ring-stage parasites were diluted to a parasitemia of 0.05% with O Rh+ erythrocytes in complete culture medium and seeded at a hematocrit of 1.5% in a total volume of 225 µl per well. Plates were subsequently incubated at 5% CO₂, 5% O₂, at 37°C before the plates were frozen and thawed three times. The growth inhibition of *Plasmodium falciparum* was measured through an enzyme-linked immunosorbent assay (ELISA) for histidine-rich protein 2 (HRP2) using a microplate reader (CLARIOstar BMG Labtech) (excitation filter: 450 nm).

Growth inhibition assays with *Plasmodium knowlesi* were performed as described previously with minor modifications. Briefly, the drugs (see Table 1) were distributed in a 3-fold serial dilution in 96-well plates. Synchronized ring-stage parasites were diluted to a parasitemia of 0.5% with O Rh+ erythrocytes and subsequently seeded at a hematocrit of 2% and complete culture medium without human AB serum but double (20%) AlbuMax II solution in a total volume of 225 µl per well. The growth inhibition of *Plasmodium knowlesi* was measured with SYBR Green-I (ThermoFisher) using CLARIOstar (BMG Labtech) (490 nm excitation filter).

*Plasmodium falciparum* and *Plasmodium knowlesi* were incubated with the drugs for 3 and 6 days to assess the onset of activity. Both time points were assessed because many antibiotics target only the progeny of the treated parasites, causing a delayed death effect that is measured only after the second replication cycle (after 6 days). Chloroquine and clindamycin were used as positive controls for 3- and 6-day assays, respectively.

### 1.4 In vitro activity against mature gametocytes of Plasmodium falciparum

Drug sensitivity assays against mature (stage V) *Plasmodium falciparum* gametocytes were performed as described previously. Briefly, the drugs were precoated in a 3-fold dilution in 96-well plates. Epoxomicin and methylene blue (Table 1) were used as positive control. Subsequently, the previously purified mature gametocytes were seeded to the plates (50,000 gametocytes/well) and incubated at 37°C in 5% CO₂ and 5% O₂. After 48 h, the ATP production was measured by the BacTiterGlo assay (Promega), according to the manufacturer's instructions. Finally, the results were quantified using a luminometer (LUmo; Autobio).

### 1.5 Thin blood smear microscopy

Asexual *Plasmodium falciparum* stages, 3D7 and Dd2 were incubated with boromycin at 1 nM for 6 h for visual observation of drug effect. Then, thin blood smears were prepared, fixed in 100% methanol for 10 seconds, and stained with Giemsa (Merck) solution (5%) for 20 minutes. The slides were washed and observed under a Leica DMBL microscope, and pictures were taken using a ProgRes C10 camera and software (Jenoptik), at 100X magnification.

### 1.6 Cytotoxicity assay

HepG2 cells (obtained from ATCC; HB-8065), a human hepatocyte carcinoma cell line, were maintained in DMEM medium (Sigma-Aldrich) supplemented with 10% of inactivated foetal bovine serum (Sigma-Aldrich), 200 mM L-glutamine (Gibco), 12 mL of HEPES buffer (Gibco), and 50 µg/ml penicillin/streptomycin solution (Gibco). Trypsin (Gibco) was used to detach the cells when they reached a semi-confluent layer.

Cytotoxicity was evaluated using the neutral red assay as described previously. Briefly, 300,000 cells were seeded in supplemented Dulbecco's Modified Eagle's Medium (DMEM) medium as described above to 96-well plates. After 24 hours, the cells were incubated with a twofold serial dilution of the respective drug diluted in supplemented DMEM medium for an additional 24 hours. Subsequently, the drug-containing medium was replaced by a supplemented DMEM medium with 1.5% of neutral red, and the cells were incubated for an additional 3 h at 37°C. Cells were then washed with phosphate buffered saline (pH 7.2), and 100 µL of freshly prepared lysing buffer (50% methanol, 49% distilled water, and 1% acetic acid) was added to the plates. Subsequently, the cells were shaken for 10 min and the absorption was measured at a wavelength of 540 nm using CLARIOstar (BMG Labtech).

### 1.7 Apicoplast as the target of boromycin

Growth inhibition assays were prepared as described in section 2.2 with modifications to assess whether the apicoplast is the main target of boromycin. Briefly, the parasites (*Plasmodium falciparum strain* 3D7) were incubated with boromycin and clindamycin (positive control) and supplemented with isopentenyl pyrophosphate trilithium salt (IPP) (Sigma) at 200 µM for 6 days as described before. The supplementation with IPP is well-described to rescue treated parasites when the apicoplast is the main target of the drug. The assay was done with and without IPP supplementation on the same plate and results were directly compared.

### 1.8 Ionophoric activity assay

Previously, boromycin was described to act as a potassium ionophore in *Bacillus subtilis* and *Mycobacterium tuberculosis*. So herein, growth inhibition assays complemented with KCI or MgCl₂ (negative control) were performed to evaluate if this is also the case in *Plasmodium falciparum.* To define a subtoxic concentration of MgCl₂ and KCI for *Plasmodium falciparum* a standard growth inhibition assays with serial dilutions of these compounds were performed as described in section 1.2. The concentration of 220 µg/mL was chosen as no signs of toxicity were seen and first inhibitory effects were observed with 440 µg/mL. Subsequently, a growth inhibition assay was prepared with a 2-fold dilution of boromycin with the addition of the sub-toxic concentration of either MgCl₂ (Sigma) or KCI (Sigma) to all wells. The assay was run in parallel to a standard growth inhibition assay of boromycin without supplementation of MgCl or KCI as control, and the obtained IC₅₀s were compared.

### 1.9. Analysis

All assays were performed at least three times in duplicates. Individual IC₅₀ values were determined by nonlinear regression analysis of log concentration-response curves, using the drc v0.9.0 package of R v2.3.1. Mean IC₅₀ values and standard deviations (SDs) were calculated for each growth inhibition assay using Excel. The chemical structures of the drugs were drawn using ChemDraw 19.0. The graphical presentation of the gametocyte inhibition was done with GraphPad Prism v7.04.

### 2. Results

### 2.1 Activity of antibiotics against asexual stages of Plasmodium falciparum and Plasmodium knowlesi

*Plasmodium falciparum* and *Plasmodium knowlesi* parasites were exposed to 15 antibiotics (tetracyclines, macrolides) and chloroquine (control drug) for 3 and for 6 days. This allowed assessing the drug's inhibitory effect on the first and second parasite replication cycle, respectively (see results in Table 2), that indicates a fast or slow onset of antiplasmodial activity of the antibiotics.

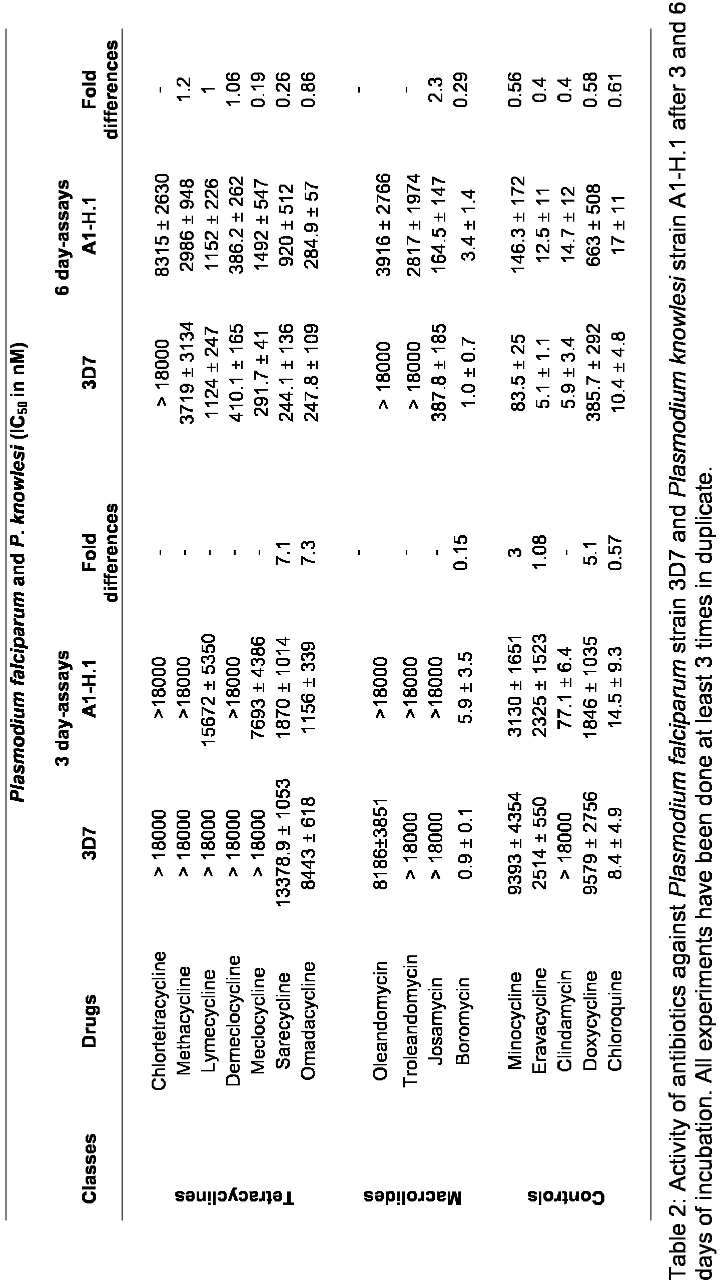

None of the tetracyclines evaluated in this study showed a pronounced activity (in the nanomolar range) in the 3-days assay against *Plasmodium falciparum* and *Plasmodium knowlesi,* but had some delayed activity after 6 days of drug exposure (Table 2). Chlortetracycline, methacycline and lymecycline were still poorly active after 6 days. The first-time tested drugs against *Plasmodium falciparum* demeclocycline, sarecycline, omadacycline and the already previously tested drugs/controls doxycycline, minocycline, and eravacycline were active in the nanomolar range against both species after 6 days (Table 2). Boromycin was highly active against *Plasmodium falciparum* and *Plasmodium knowlesi* at an IC₅₀ of 1.0 nM after 3 days of incubation (Table 2).

Most antibiotics were equipotent and displayed similar activities (within a three-fold difference) against *Plasmodium falciparum* and *Plasmodium knowlesi* especially in the 6-day assay. Differences can be seen for meclocycline, and sarecycline. In the 3-day assay *Plasmodium knowlesi* was more vulnerable than *Plasmodium falciparum* except for boromycin and oleandomycin.

### 2.2 Cytotoxicity of boromycin against human cells

Boromycin was cytotoxic at millimolar concentrations against human HepG2 cells with a selectivity index (SI) higher than 200,000. (Table 3). Chloroquine was used as comparator and was more cytotoxic than boromycin.

**Table 3: Cytotoxicity of boromycin and the comparator chloroquine against HepG2 cells and calculated selectivity index. All experiments have been done at least 3 times in duplicate.**

| | **IC₅₀ (µM) in HepG2 cells** | **SI 3D7 *** | **SI A1-H.1 *** |
|---|---|---|---|
| **Boromycin** | 1250.5 ± 230 | 1,388,000 | 211,864 |
| **Chloroquine** | 153.4 ± 52.9 | 18,214 | 10,551 |

### 2.3 Characterization of the antiplasmodial activity of boromycin

### 2.3.1 Activity of boromycin against asexual stages of Plasmodium falciparum chloroquine-resistant strains

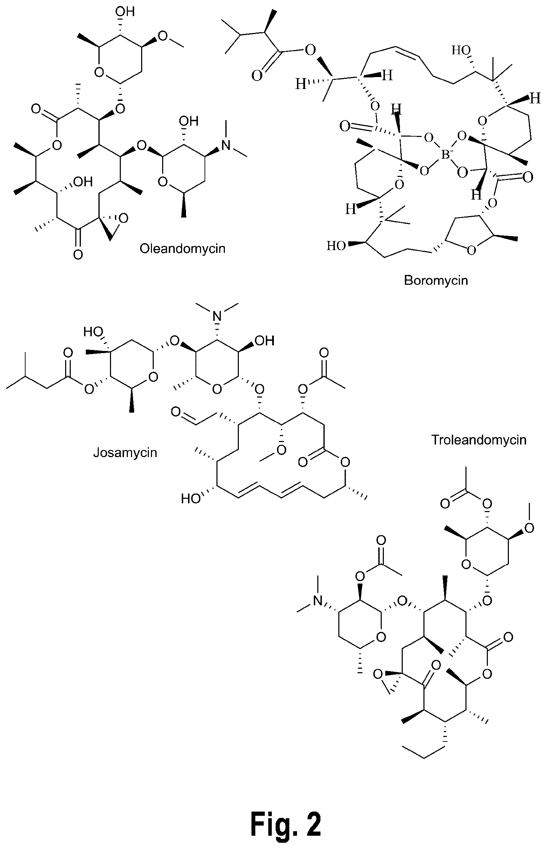

Different drug resistant strains of *Plasmodium falciparum* (Dd2, K1 and 7G8) were incubated with boromycin for 3 days to assess the potential of cross-resistance. Activities were comparable to activities against 3D7 with IC₅₀ values in the picomolar range, and no cross-resistance of boromycin in these parasite lines was seen (Table 4) (Figure 2).

**Table 4: Antiplasmodial activity of boromycin against different Plasmodium falciparum chloroquine-resistant strains in comparison to 3D7 after 3 days of incubation. All experiments have been done at least 3 times in duplicate.**

| ***Plasmodium falciparum* strains (IC₅₀ in nM)** | | | | |
|---|---|---|---|---|
| | **3D7** | **Dd2** | **K1** | **7G8** |
| **Boromycin** | 0.9 ± 0.1 | 0.6 ± 0.4 | 0.7 ± 0.1 | 0.4 ± 0.07 |
| **Chloroquine** | 8.4 ± 4.9 | 302 ± 119 | 401.4 ± 28.3 | 303.6 ± 18.2 |

In addition, *Plasmodium falciparum* parasites strain 3D7 (Figure 2 B) and Dd2 (Figure 2 C) showed the same pyknotic appearance after boromycin incubation at 1 nM for 6 h, while the untreated control showed the usual ring shape (Figure 2 A).

### 2.3.2 Activity of boromycin against mature gametocytes

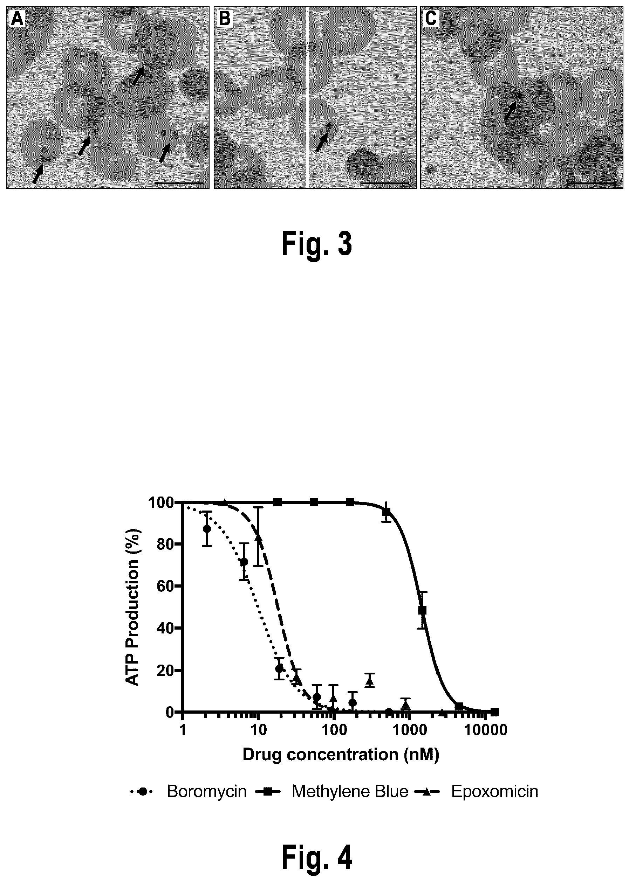

Mature *Plasmodium falciparum* gametocytes (stage IV and V) were incubated with a serial dilution of boromycin for 48 h. Afterwards, ATP production was quantified to evaluate the gametocytocidal effect (Figure 4). The results revealed that boromycin was highly active with IC₅₀ at 8.5 ± 3.6 nM, similar or even more active than the comparator compounds epoxomicin (15.2 ± 3.4 nM) and methylene blue (284 ± 35 nM), respectively.

### 2.3.3 Activity of boromycin on the apicoplast

*Plasmodium falciparum* parasites were incubated with boromycin supplemented with IPP for 6 days to assess whether boromycin targets the apicoplast as described for many antibiotics; clindamycin was used as positive control. At the same time a standard growth inhibition assay without IPP was carried out as a comparator. As expected, clindamycin-treated parasites could be rescued by the supplementation with IPP since the IC₅₀ values of control and IPP-supplemented parasites were 6.9 nM ± 1.3 and >18000 nM, respectively. In contrast to this, the IC₅₀ values for boromycin remained similar at low nanomolar concentrations for control (0.9 nM ± 0.1) and in the presence of IPP (1.4 nM ± 0.8), suggesting that this drug effect cannot be rescued by IPP and has a different target from the apicoplast.

### 2.3.4 Ionophoric activity of boromycin

*P. falciparum* parasites were incubated with a serial dilution of boromycin, and chloroquine (control) with or without supplementation of 220 µg/mL of MgCl₂ or KCI, for 3 days to assess whether boromycin has the ionophoric activity on *Plasmodium falciparum* as described for *Mycobacterium* and *Bacillus subtilis* (Table 5).

**Table 5: Activity of boromycin and the control drug chloroquine against the Plasmodium falciparum strain 3D7 in the presence/absence of KCI (220 µg/ml) and MgCl₂. (220 µg/ml). All experiments have been done at least 3 times in duplicate.**

| | IC₅₀ (in nM) | | |
|---|---|---|---|
| | control | KCl supplemented | MgCl₂ supplemented |
| Boromycin | 0.7 ± 0.1 | 2.1 ± 0.2 | 0.2 ± 0 |
| Chloroquine | 12 ± 0.8 | 11.6 ± 1.4 | 2.2 ± 0 |

Boromycin and chloroquine displayed the expected IC50 value, the supplementation of KCI at 220 µg/mL increased IC₅₀ of boromycin by 3-fold, while no difference was observed for chloroquine. The presence of MgCl2 had a synergistic effect for both boromycin and chloroquine leading to a 3-5-fold decrease in the respective IC₅₀s.

### 3. Discussion

Antibiotics for malaria treatment have been investigated since the middle of the last century, with tetracyclines playing a central role. In recent years, macrolides have also been particularly studied as antimalarials. Although the delayed death effect caused by most antibiotics limits their use as first-line chemotherapy, some antibiotics such as doxycycline, clindamycin, and the combination sulfadoxine-pyrimethamine (that does not display the delayed activity) are recommended as prophylaxis or treatment in certain populations. The need for new alternative therapies, along with the limited investments in drug discovery to treat malaria, make drug repurposing a worthy alternative. By the inventors the *in vitro* antiplasmodial activity of tetracyclines and macrolides not previously tested against Plasmodia along with comparator drugs were evaluated against blood-stages of *Plasmodium falciparum* and *Plasmodium knowlesi,* revealing boromycin, as a potent antiplasmodial drug with a fast/first cycle activity that the inventors assessed further.

All tested active tetracyclines showed a delayed death effect against both *Plasmodium* species: *Plasmodium falciparum* (strain 3D7) and *Plasmodium knowlesi* (strain A.1-H1). The newly licensed tetracyclines omadacycline (licensed for treatment of bacterial pneumonia and acute skin infections) and sarecycline (licensed for treatment of acne) showed comparable activity to doxycycline. None of the newly tested tetracyclines achieved the activity of the next generation tetracyclines tigecycline and eravacycline that suggested that modifications at the C9 position on the D ring that circumvents the ribosomal protection resistance mechanism found in bacteria could be beneficial for the activity against Plasmodia. However, the structurally similar omadacycline did not show this improved activity. In a similar way, differences in activities for these three antibiotics were also seen for rickettsia species. The delayed death effect was also shown by the macrolide josamycin suggesting that it also targets the apicoplast. The tetracyclines chlortetracycline, methacycline, lymecycline, and the macrolides troleandomycin and oleandomycin were only poorly active with IC₅₀ in the micromolar range. Whereas the macrolide boromycin showed a fast onset of activity at a single-digit nanomolar concentration.

*Plasmodium falciparum* is undoubtedly responsible for most of the malaria burden, especially for most of the malaria deaths, which justifies to mainly tailor treatments to this malaria species. However, there are five additional *Plasmodium* species responsible for malaria in humans (*Plasmodium vivax*, *Plasmodium ovale wallikeri*, *Plasmodium ovale curtisi*, *Plasmodium malariae*, and *Plasmodium knowlesi*) and when elimination of malaria is the aim, all these species must be targeted. Until recently, *Plasmodium falciparum* was the only option for drug screening *in vitro* because the other human malaria parasites could not be kept in continuous *in vitro* culture. However, now *Plasmodium knowlesi* has been adapted for *in vitro* culture and its inclusion in drug screenings could give additional information on antiplasmodial activities of compounds as it is phylogenetically closer to the other human Plasmodium species. Even though previous studies have claimed that most antiplasmodial compounds are equipotent against *Plasmodium falciparum* and *Plasmodium knowlesi,* recent studies have reported different susceptibility profiles especially for the novel compounds in development. *Plasmodium knowlesi* was described as less susceptible to inhibitors of the dihydrofolate reductase, dihydroorotate dehydrogenase, and ATP4. The inventors found in the short 3-day assay a higher activity of most antibiotics against *Plasmodium knowlesi* in comparison to *Plasmodium falciparum*. This is probably due to the assay conditions, as *Plasmodium knowlesi* has a shorter erythrocytic cycle (24 hours) compared to *Plasmodium falciparum* (48 hours) and the inventors performed the short assay for both species for 3 days. Different results might also be due to the different assay conditions such as parasitemia (0.05% versus 0.5%), haematocrit (1.5% versus 2%) and mode of evaluation (protein quantification via HRP2 versus DNA quantification via 318 SYBR-Green) of the growth inhibition assays for *Plasmodium falciparum* and *Plasmodium knowlesi,* respectively. However, despite these differing conditions the results for the 6 day/multiple cycle assay were similar for eleven of the drugs indicating that most tested antibiotics are equally active against both species.

The most promising compound from the inventors' screening was the macrolide boromycin. Boromycin was isolated from a *Streptomyces antibioticus* found in a soil sample from Ivory coast and was first described in 1967. It has a potent effect against Gram-positive bacteria *in vitro,* and it was shown to be active against HIV, *Babesia* and some other pathogens. An activity against two animal-specific Plasmodia was once described in a patent filed in the US in 1973 where its activity in models of *Plasmodium berghei* in mice and *Plasmodium gallinaceum* in birds is given. However, no description of the evaluation of its *in vitro* activity, its activity against human malaria pathogens, such as *Plasmodium falciparum* and *Plasmodium knowlesi,* or its gametocytocidal activity have been described before.

In the investigation of the inventors, boromycin showed a high activity and a fast onset of action, a desired property of antimalarials to quickly reduce parasite load and avoid the disease progression to the severe form. It also showed an excellent selectivity index against HepG2 cells. A favorable toxicity profile was confirmed and encouraged a deeper investigation about the antiplasmodial activity of boromycin.

Boromycin's *in vitro* activity against laboratory strains showed superior activity as it was active at low nanomolar and even at picomolar concentrations. No cross-resistance with the tested chloroquine/multi-resistant strains (Dd2, 7G8, and K1) was seen as the IC₅₀ values were in same range as for the drug sensitive strain 3D7, suggesting that is not affected by the common resistance mechanisms. Morphologically, ring stage parasites showed a pyknotic appearance after 6 h treatment with boromycin at 1 nM, suggesting that it leads to a rapid death. The inventors additionally evaluated the activity of boromycin against mature gametocytes and could see a very potent activity superior to the positive control methylene blue, similarly active as the proteasome inhibitor epoxomicin. Drugs with transmission-blocking activity are of special interest and would be a great asset for controlling and eliminating malaria. Currently, primaquine is the only approved drug active against gametocytes but its use is restricted mainly due to its haemolysis-inducing effect in patients with glucose-6-phosphate dehydrogenase deficiency, and additionally it has to be metabolized by a liver enzyme (CYP2D6) to be active. Compounds with multi-stage activities and novel modes of action are especially desired for the next generation of antimalarial compounds.

The apicoplast is described as the main target of antibiotics that exert the delayed death effect and the fast-acting antibiotic fosmidomycin. Apicoplast-free parasites can survive and proliferate when the medium is supplemented with IPP, but this did not rescue the boromycin-treated parasites in our assay; therefore, we could rule out the apicoplast as a main target.

The ionophoric activity of boromycin, especially for K⁺ channels, was firstly described in *Bacillus subtilis* in addition to the impairment of protein, RNA, and DNA synthesis. This ionophoric activity was later confirmed in *Mycobacterium bovis*, where a rapid loss of membrane potential, reduction of intracellular ATP, and leakage of cytoplasmic proteins were induced and the external addition of a high concentration of KCI (20 mg/mL) decreased boromycin activity from 50% to 10%. To assess whether boromycin acts as a potassium ionophore also in *Plasmodium falciparum,* parasites were simultaneously incubated with boromycin together with the highest non-toxic concentration (220 µg/mL) of KCl or MgCl₂ (negative control). The inventors could not incubate *Plasmodium* parasites at a similar high ion concentration as in the *Mycobaterium* experiments, as this concentration was toxic to the parasites. The inventors identified a three-fold IC₅₀ increase with the addition of KCI, suggesting that ionophoric activity could play a role in the potent antiplasmodial activity of boromycin. However, it is most probably not the primary mode of action, as the activity remained in the low nanomolar range. Remarkably, the inventors saw a potentiation of the drug effect by addition of MgCl.

In conclusion, the inventors presented the antiplasmodial activity of different tetracycline and macrolide antibiotics against different *Plasmodium* species. The tetracyclines tested by the inventors did not show higher *in vitro* activities than previously tested tetracyclines and all displayed a slow/second cycle onset of action. The macrolide boromycin showed the most noticeable results as a fast acting and gametocytocidal compound. These results suggest that boromycin may provide for an alternative therapy for diseases caused by apicomplexan parasites, including Plasmodia.

## Claims

1. Boromycin for use in the prevention and/or treatment of malaria by inhibition of the transmission of malaria in humans.

2. The boromycin for use according to claim 1, wherein said malaria is caused by *Plasmodium falciparum*.

3. The boromycin for use according to claim 1 or 2, wherein said malaria is caused by *Plasmodium knowlesi.*

4. The boromycin for use as a gametocytocidal compound in the prevention and/or treatment of malaria in humans.

5. The boromycin for use according to claim 4 as a gametocytocidal compound against gametocytes of *Plasmodium falciparum.*

6. The boromycin for use according to claim 4 or 5 as a gametocytocidal compound against gametocytes of *Plasmodium knowlesi.*

7. A pharmaceutical composition for use in the prevention and/or treatment of malaria by inhibition of transmission of malaria in humans comprising boromycin and a pharmaceutically acceptable carrier.

8. The pharmaceutical composition for the use of claim 7 further comprising an additional active ingredient.

9. The pharmaceutical composition for the use of claim 7 or 8, wherein said malaria is caused by *Plasmodium falciparum.*

10. The pharmaceutical composition for the use of any of claims 7 to 9, wherein said malaria is caused by *Plasmodium knowlesi.*

## Patentansprüche

1. Boromycin zur Verwendung bei der Prävention und/oder Behandlung von Malaria durch Hemmung der Übertragung von Malaria beim Menschen.

2. Boromycin zur Verwendung nach Anspruch 1, wobei die Malaria durch *Plasmodium falciparum* verursacht ist.

3. Boromycin zur Verwendung nach Anspruch 1 oder 2, wobei die Malaria durch *Plasmodium knowlesi* verursacht ist.

4. Boromycin zur Verwendung als gametocytocidale Verbindung bei der Prävention und/oder Behandlung von Malaria beim Menschen.

5. Boromycin zur Verwendung nach Anspruch 4 als gametocytocidale Verbindung gegen Gametozyten von *Plasmodium falciparum.*

6. Boromycin zur Verwendung nach Anspruch 4 oder 5 als gametocytocidale Verbindung gegen Gametozyten von *Plasmodium knowlesi.*

7. Pharmazeutische Zusammensetzung zur Verwendung bei der Prävention und/oder Behandlung von Malaria durch Hemmung der Übertragung von Malaria beim Menschen, wobei die pharmazeutische Zusammensetzung Boromycin und einen pharmazeutisch akzeptablen Träger aufweist.

8. Pharmazeutische Zusammensetzung zur Verwendung nach Anspruch 7, die ferner einen zusätzlichen Wirkstoff aufweist.

9. Pharmazeutische Zusammensetzung zur Verwendung nach Anspruch 7 oder 8, wobei die Malaria durch *Plasmodium falciparum* verursacht ist.

10. Pharmazeutische Zusammensetzung zur Verwendung nach einem der Ansprüche 7 bis 9, wobei die Malaria durch *Plasmodium knowlesi* verursacht ist.

## Revendications

1. Boromycine destinée à être utilisée dans la prévention et/ou le traitement du paludisme par inhibition de la transmission du paludisme chez l'homme.

2. Boromycine destinée à être utilisée selon la revendication 1, dans laquelle ledit paludisme est causé par le *Plasmodium falciparum.*

3. Boromycine destinée à être utilisée selon la revendication 1 ou 2, dans laquelle ledit paludisme est causé par le *Plasmodium knowlesi.*

4. Boromycine destinée à être utilisée comme un composé gamétocytocide dans la prévention et/ou le traitement du paludisme chez l'homme.

5. Boromycine destinée à être utilisée selon la revendication 4 comme un composé gamétocytocide contre les gamétocytes de *Plasmodium falciparum.*

6. Boromycine destinée à être utilisée selon la revendication 4 ou 5 comme un composé gamétocytocide contre les gamétocytes de *Plasmodium knowlesi.*

7. Composition pharmaceutique destinée à être utilisée dans la prévention et/ou le traitement du paludisme par inhibition de la transmission du paludisme chez l'homme, comprenant de la boromycine et un support pharmaceutiquement acceptable.

8. Composition pharmaceutique destinée à être utilisée selon la revendication 7, comprenant en outre un ingrédient actif supplémentaire.

9. Composition pharmaceutique destinée à être utilisée selon la revendication 7 ou 8, dans laquelle ledit paludisme est causé par le *Plasmodium falciparum.*

10. Composition pharmaceutique destinée à être utilisée selon l'une quelconque des revendications 7 à 9, dans laquelle le paludisme est causé par le *Plasmodium knowlesi.*
